# EUROPEAN PATENT APPLICATION

(11) **EP 4 480 505 A1**
(43) Date of publication of application: **25.12.2024**
(21) Application number: 23180989.8
(22) Date of filing: 22.06.2023
(51) Int. Cl.: A61L 9/00, B44C 5/04, C09D 1/02, B32B 5/02, B32B 21/10, C03C 17/22, C03C 25/42, C04B 41/45, C09D 5/14, D04H 1/4218, E04F 13/00

(54) **RECOATABLE INORGANIC DECORATIVE LAMINATE SURFACE**

(71) Applicant: Depco-Trh Pty Ltd, Williamstown Victoria 3016 (AU)
(72) Inventor: Price, David, Williamstown, 3016 (AU); Giansiracusa, Joseph, Ascot Vale, 3032 (AU)
(74) Representative: Seyer & Nobbe Patentanwälte PartmbB

(57) **Abstract**

The object of the present invention is to prepare a recoatable decorative inorganic laminate surface. To achieve the objective according to the invention a laminated substrate consists of a nonwoven glass fleece impregnated with a coating consisting of predominately calcium carbonate and potassium silicate or sol-silicate. A photocatalyst and or an antimicrobial agent may be included in the coating mixture, and to increase the efficiency of the photocatalyst on the decorative laminate surface a deep UVC radiation source of 222nm wavelength is used in order to kill any pathogens entrained in the airflow and to activate the photocatalyst to destroy pollutants.

## Description

The invention relates to a recoatable decorative laminate consisting predominately of an inorganic coating on a building material substrate.

In the field of decorative laminates there are well known examples such as low pressure laminates (LPL) also known as low pressure melamine (LPM), continuous pressure laminates (CPL) and high pressure laminates (HPL). The manufacture of these well-known decorative laminates includes the use of melamine and formaldehyde. Patent application EP3896043 describes a high opacity laminate surface which includes melamine in the formulation, and includes a mineral silicate layer and a photocatalyst functioning in sunlight and indoor lighting, whereby a laminate material with a mineral silicate layer and a photocatalyst are claimed. However, the inclusion of free melamine (CAS 108-78-1) as a component of the surface coating in EP3896043 brings the commercial viability of the patent application into question as melamine has recently been added by the European Chemicals Agency (ECHA) to the Candidate List of Substances of Very High Concern (SVHC) which can result in future restrictions in the use of melamine. The limit proposed for free melamine is not more than 0.1 %. Formaldehyde (CAS 50-00-0) according to ECHA harmonised classification and labelling "this substance is toxic if swallowed, is toxic in contact with skin, causes severe skin burns and eye damage, is toxic if inhaled, may cause cancer, is suspected of causing genetic defects and may cause an allergic skin reaction".

The object of the present invention is to manufacture a recoatable inorganic decorative laminate surface suitable for indoor and outdoor applications. The surface is predominately inorganic material applied into and on a nonwoven glass fleece laminated to a building material substrate. There is no hot pressing required to manufacture the laminate, and the surface does not contain melamine or formaldehyde.

To achieve the objective according to the invention the surface coating including the carrier material is predominately inorganic. The carrier material laminated to a building material substrate is a nonwoven glass fleece and the coating material is a mixture of calcium carbonate and a liquid potassium silicate or sol-silicate. Preferably the calcium carbonate content of the coating exceeds 50% of the inorganic materials content excluding the nonwoven glass fleece. The recoatable inorganic decorative laminate surface may include pigments to achieve a particular colour, preferably inorganic pigments with a high light fastness, an inorganic antimicrobial agent for example Ishizuka Japan IONPURE glass encapsulated silver ions, an inorganic photocatalyst based on TiO₂ and a dispersing agent. The coating may be applied in more than one coating step, and any photocatalyst included in the coating is preferably concentrated in the outermost region of the coating. The photocatalyst can be activated by sunlight as well as synthetic light sources such as deep UVC 222nm radiation or a VIS light source. Advantageously the synthetic UVC 222nm radiation and VIS light source can be used when no sunlight is available. The surface of the laminate can be sanded to achieve a smooth silky feeling, and has an anti-fingerprint attribute. A clear protective coating may be applied to the surface of the laminate.

In a first example of a recoatable inorganic decorative laminate according to the invention a 70g/m² nonwoven glass fleece (Ahlstrom Finland) is laminated to a 12mm MDF panel using Selleys^{®} Allfix^{®} Big Jobs adhesive. A coating mixture is prepared consisting of 30g calcium carbonate (Langridge Marble Dust, Melbourne Art Supplies AU), 70g potassium silicate (Durobond Off-White approximately 30% solids), 17g dispersing agent styrene maleic anhydride amic acid (Xiran SL40005 N30, Polyscope NL), and 1g of black dispersion (Carbofin Schwartz LC2900, Rockwood). The resultant mixture is a dark grey colour, the solids content of the coating mixture is approximately 89% inorganic matter, and is applied to the nonwoven glass fleece on the laminated panel. The wet coating weight is 488g/m², and after 3 hours drying at ambient room temperature the weight of the coating is 298g/m². The laminate coating including the nonwoven glass fleece is approximately 91% inorganic matter.

In a second example of the coating mixture a photocatalyst is added. The solids content of the coating mixture consists of approximately 55.5% calcium carbonate, 5.5% photocatalyst and 39% potassium silicate. The materials used in making the coating were: 30g of calcium carbonate (Langridge Marble Dust, Melbourne Art Supplies AU,), 3g of photocatalyst KRONOClean 7050 (Kronos Leverkusen DE), and 70g of potassium silicate paint (Durobond Sydney AU, Off-white approximately 30% solid content). The resulting mixture was applied to piece of 5 layer 15mm plywood (Carter Holt Harvey, Myrtleford AU) that was earlier laminated with a 70g/m² nonwoven glass fleece using Selleys^{®} Allfix^{®} Big Jobs adhesive. Measurements after the coating showed the wet coating weight as 430g/m², and after drying overnight in ambient room temperature as 260g/m².

In a third example of the coating mixture, calcium carbonate is approximately 50.8% of the solids, the photocatalyst 5.1% of the solids, potassium silicate is approximately 35.6% of the solids and a dispersant approximately 8.2%. The materials used in making a coating were: 30g of calcium carbonate (Langridge Marble Dust, Melbourne Art Supplies AU,), 3g of photocatalyst KRONOClean 7050 (Kronos Leverkusen DE), 70g of potassium silicate paint (Durobond Sydney AU, Alfalfa Green approximately 30% solid content), and16g (30% solids) dispersing agent styrene maleic anhydride amic acid (Xiran^{®} SL40005 N30, Polyscope NL). The resulting mixture was applied to a piece of 18mm particleboard (Borg Manufacturing, Oberon AU) that was earlier laminated with a 70g/m² nonwoven glass fleece using Selleys^{®} Allfix^{®} Big Jobs adhesive. Measurements after the coating showed the wet coating weight as 679g/m², and after drying for 3 hours in ambient room temperature as 402g/m². After a light sanding with Grit 600 sandpaper the weight is 375g/m².

A fourth example was prepared by applying the coating mixture of example 3 to a 6mm OSB panel laminated with a 70g/m² nonwoven glass fleece. The wet coating weight was 412g/m², and after 3 hours in ambient room temperature weighed 211g/m². Overnight the coating weight reduced to 184g/m². A second coating was applied with a wet coating weight of 237g/m². After 3 hours ambient room temperature drying the coat weight was 144g/m². After a light sanding with Grit 600 sandpaper the weight was 137g/m² and the surface has a smooth and silky feeling.

A fifth example was prepared by taking the coated MDF panel of example 1 and coating the surface with a clear potassium silicate (Durobond Clear Silicate Matte). The wet clear potassium silicate coating weighed 125g/m². After 3 hours ambient room temperature drying the coat weight was 11.2g/m².

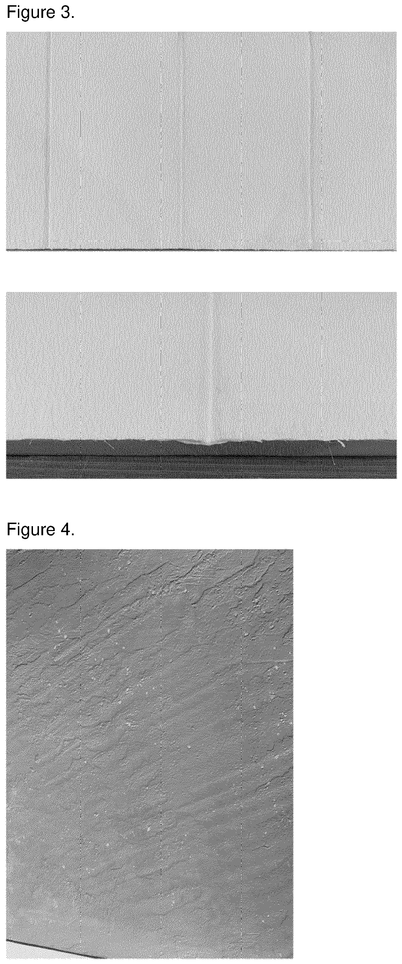

A sixth example was prepared with a fire retardant intermediate layer between the substrate and the nonwoven glass fleece. The substrate was 12mm MDF and the intermediate layer was a 200g/m² paper (FHB1 JagoTechPaper DE), laminated to the MDF surface using Selleys^{®} Allfix^{®} Big Jobs adhesive. Subsequently, a 70g/m² nonwoven glass fleece was laminated to the panel using the same adhesive and coated with the inorganic mixture of example 3. The wet coat weight was 893g/m² and after drying the weight was 597g/m2. This large amount of the coating mixture enabled a texture to be created on the surface, see Figure 4. The coated panel was lightly sanded with Grit 600 sandpaper removing approximately 10g/m² of coating material and then a clear protective layer of potassium silicate applied. The wet amount of the protective layer of potassium silicate was 144g/m2., and after drying 16g/m².

The source of calcium carbonate is not limited, however it is preferred that the calcium carbonate is the waste "marble dust" recovered from the mining and processing of marble deposits. Other sources of calcium carbonate may also be used including dolomite (Ca Mg (CO₃)₂), and when available in commercial quantities biogenic CaCOs such as shells or coral. It is also preferred that when a photocatalyst is used, the calcium carbonate powder and the photocatalyst powder are mixed together before being dispersed in the liquid potassium silicate or sol-silicate paint.

There is no limit to the hue or weight of the nonwoven glass fleece, for example Ahlstrom Finland nonwoven glass fleece is available with a white appearance or a black appearance, available from 20g/m² to 120g/m². The 70g/m² used in producing the examples is referred to as white glassfibre tissue GFT-25K13-70.

There may be more than one coating of the inorganic mixture applied to the nonwoven glass fleece and preferably, when a photocatalyst is included in the coating, the majority of the photocatalyst is in the outermost region of the coating layer. Furthermore, there may be an intermediate sanding of any individual layer, and optionally a silicate primer applied before the next coating. There may be more than one material layer, and a material layer may contain performance enhancing additives such as fire retardants. The surface of the recoatable inorganic decorative laminate may be printed or have an abstract motif applied and, whether printed or not, may have a clear protective layer. To maintain access to the photocatalyst a clear protective layer can be an unpigmented non-film forming potassium silicate, alternatively the clear potassium silicate protective layer may contain a photocatalyst. When there is no photocatalyst or antimicrobial additive in the inorganic coating, a closed surface coating such as Crommelin Diamond Coat^{®} or Dulux^{®} Clear Top Coat may be used. For example a coating of Crommelin Diamond Coat^{®} Satin was applied to example 3, measuring as a wet coating 144g/m², and later after drying overnight measuring 67.4g/m².

Further advantageous embodiments according to the invention result from the sub-claims.

A recoatable inorganic decorative laminate coating containing a photocatalyst allows the use of devices emitting UVC 222nm radiation. By having room airflow through the UVC 222nm radiation zone and onto the surface containing the photocatalyst, there is an increase in the effectiveness in cleaning the air and surfaces in a room. While exposure to UV radiation can cause damage to human skin and eyes, studies available in the public domain have shown that deep UVC 222nm radiation is less harmful to humans thereby providing under appropriate circumstances a safe method for cleaning of the air without having to operate the UVC 222nm radiation device in an enclosed system. Recommendations for radiation exposure limits can be found in IEC 62471 "Photobiological Safety of Lamps and Lamp Systems". The scope of this standard is to provide guidance for the evaluation of the photobiological safety of lamps and lamp systems, excluding lasers, that emit light in the spectral region 200-3000 nm.

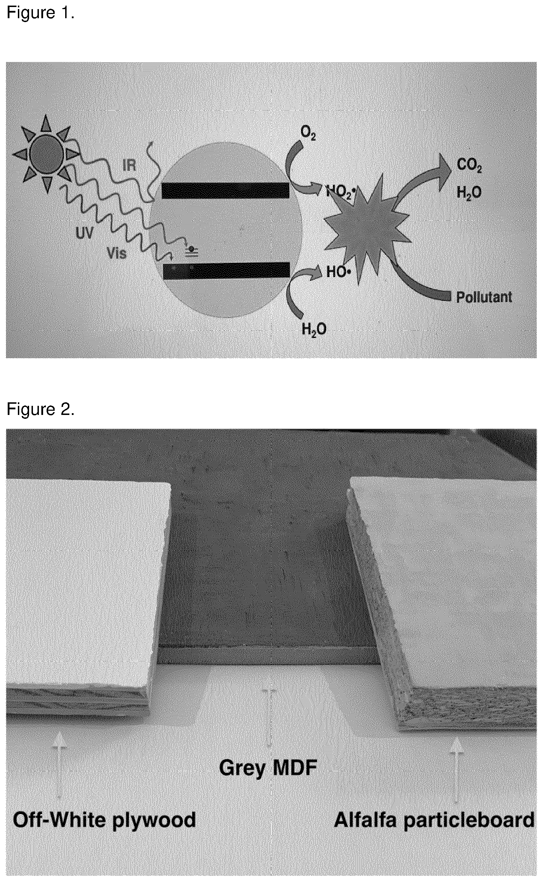

An example of how a photocatalyst is activated is shown in Figure 1.

In a first aspect of the invention at least part of an existing wall and/or ceiling in a room are laminated with a nonwoven glass fleece using a suitable adhesive such as Selleys^{®} Allfix^{®} Big Jobs adhesive. A coating is applied to the nonwoven glass fleece being a mixture of calcium carbonate and liquid potassium silicate or sol-silicate paint.

In a second aspect of the invention new wall panels and or new ceiling panels are first laminated with a nonwoven glass fleece, and then a coating containing calcium carbonate and potassium silicate or sol-silicate is applied to form at least part of a wall or ceiling structure. The substrate for the coating may be freely chosen from a variety of building materials, for example the substrate may be plasterboard, solid wood or a wood based panel, or a rigid XPS extruded panel. It would be possible to use as a substrate a high pressure laminate (HPL), continuous pressure laminate (CPL) or compact laminate, or a low pressure melamine panel (LPM). Thin and lightweight panels such as HPL and CPL have an inherent handling advantage in that they may be easily attached, removed and reattached to a wall by using a non-invasive method such as Velcro^{®} strips.

Preferably the new panels are made using an environmentally friendly substrate, for example a wood based panel such as medium or high density fibreboard, particleboard, oriented strand board, plywood or cross laminated timber, preferably manufactured without a formaldehyde binder, or if a formaldehyde binder is used the emission level should not exceed E1 according to EN13896. The wood panel surface may be smooth or have a recessed area, such as a V-groove, see Figure 3. There may be an additional material layer applied to the substrate before the nonwoven glass fleece is applied, and the additional material layer may contain performance enhancing components, for example fire retarding additives. The decorative laminate coating may be applied in more than one application and may include a primer between coatings, for example a primer used with a potassium silicate paint (Porter's Paints AU, Mineral Silicate Primer, containing 1-10% potassium silicate).

The reverse side of any laminated substrate may have a functional material coating or layer such as but not limited to heat reflection, electromagnetic radiation reflection, a moisture barrier, an airtight barrier, sound dampening or any combination of these functional objectives. An example of material used as the reverse layer is an expanded polystyrene (EPS) material laminated with a thin aluminium metal sheet (Climapor^{®}, Saarpor Klaus Eckhardt GmbH DE). Preferably the EPS is fire retardant, for example Neopor^{®} F (BASF).

In a third aspect of the invention, a renovation of the recoatable inorganic decorative laminate surface can be made in-situ as and when required. If necessary the panel may be removed from the room and repaired off-site. The original decorative laminate coating may be lightly sanded and recoated with the inventive coating, or a re-coating that does not use the inventive coating. The re-coating of the laminate may be for example a potassium silicate paint containing a photocatalyst and or an antimicrobial agent. The renovation of a panel should consider environmental aspects and where feasible and financially reasonable the panel repaired, refurbished or recycled. The panel with the inventive coating may feature a commercial advertisement, or an industrial design or be a unique art piece of considerable value. Preferably, the pigments used in achieving a colour, or an abstract design, or a printed motif are lightfast inorganic pigments.

In a fourth aspect of the invention, a material layer of nonwoven glass fleece is impregnated with the inventive coating before being laminated to a surface, a panel or a sheet. The impregnation and or coating of the nonwoven glass fleece may be a partial impregnation and a final coating applied after application of the nonwoven glass fleece layer to a surface, panel or sheet.

In a fifth aspect of the invention, the TiO₂ photocatalyst in powder form is mixed with the calcium carbonate in powder form to improve distribution and reduced agglomeration of the TiO₂ photocatalyst when added to the liquid potassium silicate or sol-silicate paint. A dispersing agent may be added when the powders are mixed, or when the powders are added to the liquid potassium silicate.

The inventive recoatable inorganic decorative laminate can be used for interior applications, such as in a room or a large interior open space including as wall panels, ceiling panels, door panels, room divider panels, pieces of furniture or on functional equipment such as a radiator panel. When a protective coating suitable for exterior use is applied to the laminate surface, and the building substrate is qualified by the manufacturer for exterior use, then the recoatable inorganic decorative laminate may be used for some exterior applications.
Fig.1 schematic of how a photocatalyst works
Fig.2 show examples of the recoatable inorganic decorative laminate
Fig.3 shows a recoatable inorganic decorative laminate using a wood based panel with V-grooves

## Claims

1. A recoatable decorative laminate comprising a surface with a mixture of inorganic materials applied into and on a nonwoven glass fleece in one or more coating steps, **characterised in that** the inorganic materials are predominately calcium carbonate and potassium silicate.

2. A decorative laminate surface according to claim 1, wherein the mixture contains an inorganic photocatalyst, and/or inorganic antimicrobial agent.

3. A decorative laminate surface according to claims 1 - 2, wherein the mixture contains a dispersing agent.

4. A coated surface according to claim 1, wherein the calcium carbonate is marble dust recovered from the mining and processing of marble slabs including dolomite, and/or wherein the calcium carbonate is from a biogenic source.

5. A surface, panel or sheet according to claims 1 - 4, wherein the surface, panel or sheet is laminated with a nonwoven glass fleece and subsequently a coating according to the invention is applied, and/or wherein the surface is a panel or a sheet and has at least on one side a predominately inorganic coating covering part or all of the surface.

6. A surface, panel or sheet substrate according to claims 1 - 5, wherein the nonwoven glass fleece is partially or fully impregnated with the inorganic coating prior to being attached to the surface, panel or sheet.

7. A surface, panel or sheet substrate according to claims 1 - 6, wherein there is an intermediate layer with fire retardant additives located between the nonwoven glass fleece and the substrate.

8. A recoatable inorganic decorative laminate according to claims 1 - 7, wherein the surface is unicolour, or printed, or has an abstract design, and/or wherein the surface is smooth or has a structure.

9. A recoatable inorganic decorative laminate according to claim 8, wherein the surface has a clear protective coating, and/or wherein the re-coating of the surface is a potassium silicate paint.

10. A recoatable inorganic decorative laminate according to claims 9, wherein the clear protective coating or the re-coating contains a photocatalyst and or an antimicrobial agent.

11. A panel or sheet used as a substrate for a decorative laminate according to the invention, wherein the panel or sheet is a plasterboard, or a wood based panel, or a rigid extruded XPS panel, or a high pressure laminate (HPL), or a continuous pressure laminate (CPL), or a compact laminate, or a low pressure laminated wood based panel (LPM).

12. A wood based panel according to claim 11, wherein the wood based panel has milled recesses in the surface such as V-grooves.

13. A panel or sheet according to claim 11-12 used as a substrate, wherein the reverse side of the panel or sheet has a functional material coating such as but not limited to heat reflection, electromagnetic radiation reflection, a moisture barrier, an airtight barrier, sound dampening or any combination of these functional objectives.

14. A method for reducing pollutants and pathogens in indoor air and on indoor surfaces, **characterized by** directing room airflow onto a decorative laminate surface as described in claim 2, whereby simultaneous irradiating of the photocatalyst in the surface with radiation from a deep UVC radiation source of 222nm wavelength takes place in order to kill any pathogens entrained in the airflow and activate the photocatalyst to destroy pollutants.

15. A method for removing pollutants and pathogens from indoor air and on indoor surfaces according to claims 9, wherein the UVC 222nm radiation device is manoeuvred by a programmable apparatus to change the direction of radiation regularly or continuously thereby broadening the amount of surface with the photocatalyst being exposed to UVC 222nm radiation and simultaneously preventing the radiation being concentrated on one particular position of the decorative laminate surface.
